# EUROPÄISCHE PATENTSCHRIFT

(11) **EP 0 523 449 B1**
(45) Veröffentlichungstag und Bekanntmachung des Hinweises auf die Patenterteilung: **15.05.1996**
(21) Anmeldenummer: 92111142.3
(22) Anmeldetag: 01.07.1992
(51) Int. Cl.: C07K 5/06, C07C 271/22

(54) **Reduktive Aminierung einer Aminosäure oder eines Aminosäure-Derivates mit einer Alpha-Ketosäure oder einem Alpha-Ketosäurederivat**
Reductive amination of an aminoacid or an aminoacid derivative with an alpha-keto acid or an alpha-keto acid derivative
Amination réductive d'un acide aminé ou d'un dérivé d'acide aminé par un alpha-cétoacide ou un dérivé d'un alpha-cétoacide

(30) Priorität: 13.07.1991 DE 4123248
(43) Veröffentlichungstag der Anmeldung: 20.01.1993
(73) Patentinhaber: DEGUSSA AG, D-60311 Frankfurt (DE)
(72) Erfinder: Kottenhahn, Matthias, Dr., W-6450 Hanau 7 (DE); Harr, Horst, W-6458 Rodenbach (DE); Drauz, Karlheinz, Dr., W-6463 Freigericht 1 (DE)

(56) Entgegenhaltungen:
- US-A- 4 474 692
- JOURNAL OF MEDICINAL CHEMISTRY Bd. 28, Nr. 11, 1985, Seiten 1596 - 1602 JOHNSON, A.L. ET AL. 'Synthesis and pharmacology of the potent ...'
- CHEMISTRY LETTERS 1988, Seiten 1691 - 1694 IWASAKI, G. ET AL. 'A
- stereoselective synthesis ...'

## Beschreibung

Die Erfindung betrifft eine reduktive Aminierung einer Aminosäure oder eines Aminosäurederivates mit einer α-Ketosäure oder einem α-Ketosäurederivat in einem inerten Lösungsmittel in Gegenwart eines Hydrierkatalysators und Hydriermittels sowie gegebenenfalls unter Wasserentfernung und insbesondere ein Verfahren zur Herstellung von Verbindungen der Formel 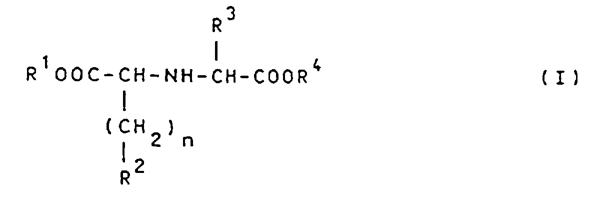 in welcher
- R¹: Wasserstoff, Methyl, Ethyl oder Benzyl,
- R: Wasserstoff, C₁-C₄-Alkyl, Phenyl oder Phenyl-[C₁-C₄-alkyl]
- R³: Wasserstoff, Heteroalkyl- oder C₁-C₄-Alkyl- Benzyl-, 4'-Hydroxybenzyl-, Acylamino-[C₁-C₅]-alkyl-, tert. Butyloxycarbonylamino-[C₁-C₅]-alkyl-, Benzyloxycarbonylamino-[C₁-C₅]-alkyl-
- R⁴: H oder eine Esterschutzgruppe,
- n: 0, 1 oder 2
bedeuten, durch hydrogenolytische Umsetzung einer Verbindung der Formel 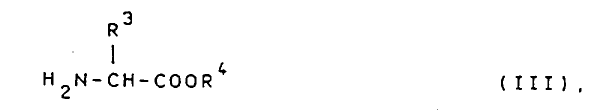 in der R³ und R⁴ eine der bereits angegebenen Bedeutungen aufweisen,
mit einer Verbindung der Formel

R-(CH₂)ₙ-CO-COOR¹ (V),

in der R¹, R und n eine der bereits angegebenen Bedeutungen aufweisen,
oder daß Verbindungen der Formel 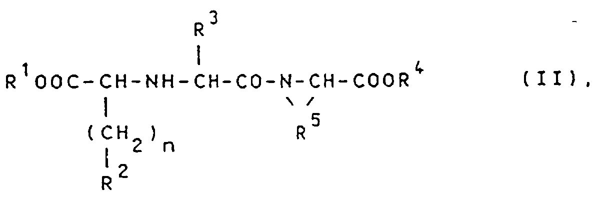 in welcher R¹, R, R³, R⁴ und n die oben angegebene Bedeutung haben,
- R⁵: zusammen mit den sie tragenden Atomen ein heterocyclisches mono- oder bicyclisches Ringsystem mit 5 bis 10 Ringgliedern bedeutet,
durch hydrogenolytische Umsetzung einer Verbindung der Formel 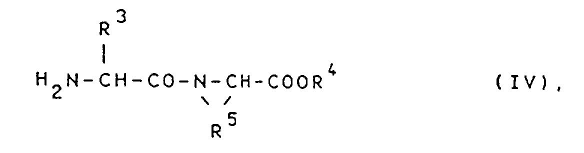 in der R³ bis R⁵ eine der bereits angegebenen Bedeutungen aufweisen, mit einer Verbindung der Formel

R-(CH₂)ₙ-CO-COOR¹ (V),

in der R¹, R und n eine der bereits angegebenen Bedeutungen aufweisen, in Gegenwart eines inerten Lösungsmittels, eines Hydrierkatalysators, eines Hydriermittels und gegebenenfalls unter Entzug von Wasser, erhalten werden. Grundsätzlich schließen "Alkyl", "Acyl", "Aromat", etc. bei den Resten R auch entsprechende Reste mit typischen Substituenten ein, z. B. 4-(Trifluoracetylamino)-butyl für R³.

N-substituierte α-Aminosäuren (wie I) und diese enthaltende Dipeptide (wie II) sind wertvolle Zwischenprodukte für die Herstellung von Inhibitoren des Angiotensin Converting Enzymes (ACE), die als Blutdruckregulatoren hochinteressant sind. Einige solche N-substituierte Dipeptide sind bereits als Arzneimittel im Handel oder sie befinden sich im Stadium der Zulassung,
beispielsweise Enalapril (N^{α}-[(S)-1-Ethoxycarbonyl-3-phenylpropyl]-L-alanyl-L-prolin), Lisinopril (N^{α}-[(S)-1-Carboxy-3-phenylpropyl]-L-lysyl-L-prolin) oder Ramipril (2-[N^{α}-[(S)-1-Ethoxycarbonyl-3-phenylpropyl]-L-alanyl] -(1S, 3S, 5S)-2-azabicyclo[3,3,0]octan-3-carbonsäure).

Ein derartiges Verfahren ist in J.Org.Chem.1988,53, 836-844 für die Herstellung von Verbindungen der Formel (II) beschrieben. Als inertes Lösungsmittel dient absolutes Ethanol, zur Wasserentfernung ein Molekularsieb und als Hydrierkatalysator Raney-Nickel. Die hierbei entstehenden Produkte werden in 42 - 80 % Ausbeute gemäß der Literatur erhalten. Diese Ausbeuten können jedoch schwer reproduziert werden, zudem sind in den Produkten meist noch erhebliche Mengen an Nebenprodukten enthalten, die die Aufreinigung zu pharmazeutisch verwendbaren Produkten erschweren.

In J. Med. Chem. 28 (1985), 1596 - 1602 wird eine reduktive Aminierung eines Aminosäureesters mit Brenztraubensäure beschrieben. Der Aminosäureester wird als Hydrochlorid eingesetzt. Im Lösungsmittel wird eine geringe Menge NaOH gelöst, das der Freisetzung des Amins aus dem Aminosäureester-Hydrochlorid und der teilweisen Neutralisation der Brenztraubensäure dient. Insgesamt liegt im Reaktionsgemisch, bedingt durch die Brenztraubensäure, ein Säureüberschuß vor.

Aus der US-PS 4,474,692, insbesondere Beispiele 2, 7 - 14 und 21 sind weitere reduktive Aminierungen von Dipeptiden mit α-Ketosäuren bekannt. Im Reaktionsgemisch ist ein Puffer aus Natriumacetat und Essigsäure enthalten, in dem die Essigsäure im ungefähr doppelten molaren Überschuß vorliegt.

Aufgabe der Erfindung ist eine Modifizierung der bekannten Verfahrens für hohe Ausbeuten bei weniger Nebenprodukte, wobei keine hohen zusätzlichen Kosten entstehen sollen. Außerdem soll die Reaktion - sofern gewünscht - im Hinblick auf die Bildung der Diastereomeren beeinflußbar sein.

Diese Aufgabe wird bei dem erfindungsgemäßen Verfahren dadurch gelöst, daß man die Umsetzung in Gegenwart eines Basenüberschusses einer organischen oder anorganischen Base vornimmt.

Ein Basenüberschuß ist dann bezüglich des Gesamtsystems gegeben, wenn während der kompletten Reaktion das molare Verhältnis aus Basenfunktionen und Säurefunktionen > 1 ist.

Durch diesen Überschuß wird überraschenderweise die chemische Ausbeute der Umsetzung deutlich erhöht, obwohl der erste Schritt der Reaktion, die Iminbildung (Roberts, J. D.; Caserio, M. C.; Basic Principals of Organic Chemistry, 2. Ed., W. A. Benjamin Inc. Philippines, 1977, S. 697, 1154), säurekatalysiert abläuft. Besonders geeignet sind schwache Basen, z. B. organische Basen wie Mono-, Di- oder Triethanolamin, Trimethylamin, Triethylamin, Tripropylamin, Tributylamin oder N-Methylmorpholin.

Geeignete anorganische Basen sind z.B. Salze schwacher Säuren wie NaOAC, K₂CO₃, KHCO₃, Na₂CO₃ NaHCO₃ und, besonders bevorzugt, basisches Aluminiumoxid.

Starke Basen können insbesondere in sehr verdünnter Konzentration ebenfalls die Reaktion unterstützen.

Die Base wird zweckmäßigerweise in einer Menge von 0.01 bis 5 Moläquivalenten, vorzugsweise von 0,05 bis 2 Moläquivalenten, jeweils bezogen auf die eingesetzte Verbindung der Formel (III) oder (IV), zugesetzt. Bei der Verwendung organischer Basen sind unterstöchiometrische Mengen, insbesondere 0,08 - 0,6 Moläquivalente besonders bevorzugt.

Die Aminofunktion der eingesetzten Aminosäuren oder Aminosäurederivate muß während der Reaktion zumindest zum Teil in Form eines primären Amins vorliegen, dies ist bei dem erfindungsgemäßen Verfahren auch gegeben, wenn diese Verbindung als inneres Salz oder sonst als Salz, z. B. als Hydrochlorid, eingesetzt werden.

Das Aminosäurederivat ist vorzugsweise ein Ester der Aminosäure oder ein Amid der Säurefunktion, wobei die amidische Bindung bevorzugt mit einer weiteren Aminosäure oder -derivat erfolgt, so daß das Aminosäurederivat ein Di- oder Oligopeptid ist. Insgesamt ist die Verwendung von α-Aminosäuren bzw. deren Derivate bevorzugt.

Als α-Ketosäuren oder deren Derivate sind besonders Verbindungen der Formel V bevorzugt, wobei Brenztraubensäure der einfachste Vertreter ist.

Meist erfordert die Reaktion die Entfernung des entstehenden Wassers, ein verwendetes Wasserbindemittel ist vorzugsweise inert oder basisch. Geeignete Wasserbindemittel sind z. B. Molekularsiebe und Aluminiumoxide. Der Wasserentzug ist insbesondere bei der Reaktion der Ketogruppe mit dem Amin zum Imin vorteilhaft und vor allem beim Einsatz von α-Ketoestern angezeigt, da sonst unter Umständen auch die Reduktion der Ketofunktion als Nebenreaktion verstärkt stattfindet.

Der Zusatz der organischen Base macht es häufig möglich, die als Wasserbindemittel eingesetzte Menge an Molekularsieb deutlich zu verringern, der Zusatz eines basischen Aluminiumoxids macht das Molekularsieb unter Umständen ganz entbehrlich, weil es selbst gleichzeitig als Wasserbindemittel dient. Dies führt neben einer Kostenersparnis vor allem zu weniger Abfallprodukten.

Wird als Zusatzstoff eine anorganische Base, z.B. ein basisches Aluminiumoxid eingesetzt, dann geschieht dies zweckmäßigerweise in einer Menge von 2 g/mol bis 400 g/mol, bezogen auf die eingesetzte Verbindung der Formel (III) oder (IV), beziehungsweise, wenn mit dieser Base gleichzeitig das gebildete Reaktionswasser gebunden werden soll, in Mengen von 50 bis 500 g/mol.

Die verwendeten Lösungsmittel sollen vorzugsweise wasserfrei sein, für das erfindungsgemäße Verfahren geeignete inerte Lösungsmittel sind Alkohole, wie Methanol, Ethanol, Propanol oder Butanol; Ether, wie Ethylenglykoldimethylether, Diethylenglykoldimethylether oder Tetrahydrofuran; und Carbonsäuredialkylamide, wie Dimethylformamid oder Dimethylacetamid. Auch Gemische solcher Lösungsmittel können eingesetzt werden. Vorteilhaft wird zumindest ein protisches Lösungsmittel eingesetzt.

Als Hydrierkatalysatoren und Hydriermittel werden bevorzugt Raney-Nickel, Palladium oder Palladium auf Aktivkohle und Platin oder Platin auf Aktivkohle sowie H₂ eingesetzt. Wenn keine Stereoselektivität gewünscht wird, können auch Hydride als Hydrierkatalysatoren und Hydriermittel eingesetzt werden. Raney Nickel ist besonders bevorzugt, da die Diastereoselektiviät hier am besten ist.

Von den bei der Reaktion entstehenden Diastereomeren ist meist das S-konfigurierte Produkt bevorzugt. Die zur bevorzugten Herstellung des Enantiomeren erforderliche Diastereoselektivität der Umsetzung kann in vielen Fällen noch erhöht werden, wenn man eine chirale organische Base verwendet. Solche Basen sind beispielsweise L- oder D-Prolinol, L- oder D-Valinol, (-)- oder (+)-Ephedrin und (-)- oder (+)-Norephedrin, die je nach den beteiligten Verbindungen oft die Bildung eines Enantiomeren fördern.

Bei der Herstellung von Verbindungen der Formel (I) werden als Verbindungen der Formel (III) bevorzugt die natürlich vorkommenden α-Aminosäuren oder deren optische Antipoden, gegebenenfalls in geschützter Form, eingesetzt. Beispiele sind Alanin, Valin, Methionin, Cystein, Serin, Tryptophan, Asparagin, Glutamin, Asparaginsäure, Glutaminsäure, Phenylalanin, Tyrosin, Leucin, Isoleucin oder Glycin, und deren Ester, zum Beispiel die Benzyl-, tert. Butyl- oder Ethylester (Esterschutzgruppen). Wird als Verbindung der Formel (III) Lysin oder Ornithin eingesetzt, so muß die seitenkettigständige Aminogruppe geschützt werden, beispielsweise durch eine Trifluoracetyl-, Carbobenzoxy- oder tert. Butyloxycarbonylgruppe.

Bei der Umsetzung von Verbindungen der Formel (III) mit einer Verbindung der Formel (V) kann es vorteilhaft sein, wenn man diese zunächst zusammengibt und mit Hilfe eines geeigneten wasserschleppenden Lösungsmittels, beispielsweise Cyclohexan, Methyl-tert. butylether oder Toluol, unter Wasserabscheidung erhitzt und so azeotrop entwässert. Dabei können die Verbindungen der Formel (III) wie auch der Formel (II) gegebenenfalls auch in Form von Salzen, zum Beispiel als Paratoluolsulfonate oder Hydrochloride, eingesetzt werden, wenn R⁴ nicht H bedeutet. Zur Überführung der Salze in ihre freien Amine sollte schon bei der azeotropen Entwässerung die Base in einem Molverhältnis bis zu 100 mol-% vorliegen. Bevorzugt sind hierbei die schwachen Basen wie N-Methylmorpholin. Bei dieser Vorgehensweise muß darauf geachtet werden, daß die Bildung von Lactonen oder Dimerisierungsprodukten der Verbindung (V) vermieden wird, hohe Temperatur (insbesondere über 100^{o}C) sollten entsprechend vermieden werden. Anschließend wird gegebenenfalls das Lösungsmittel gewechselt und hydrogenolytisch umgesetzt, d. h. die Base und der Hydrier-Katalysator werden zugegeben.

Bei der Herstellung von Verbindungen der Formel (II) werden als Verbindungen der Formel (IV) bevorzugt Dipeptide aus einer natürlich vorkommenden α-Aminosäure oder deren Antipoden, in einer etwaigen Seitenkettenfunktionalität gegebenenfalls geschützt, in N-terminaler Position und einer cyclischen α-Aminosäure in C-terminaler Position eingesetzt. Beispiele sind Alanyl-prolin, N^{ε}-Trifluoracetyl-lysylprolin, N^{ε}-Carbobenzoxy-lysyl-prolin, N^{ε}-tert.-Butyloxycarbonyl-lysyl-prolin, N^{δ}-Trifluoracetyl-ornithyl-prolin, N^{δ} -Carbobenzoxy-ornithyl-prolin oder N^{δ}-tert.Butyloxycarbonyl-ornithyl-prolin.

Als Verbindung der Formel (V) wird bevorzugt 2-Oxo-4-phenyl-buttersäureethylester verwendet. Die Verbindungen der Formel (V) werden zweckmäßigerweise in einem 0,05 bis 1-fachen molaren Überschuß über die jeweilige Verbindung der Formel (III) oder (IV) eingesetzt.

Bei der praktischen Durchführung des erfindungsgemäßen Verfahrens wird eine Verbindung der Formel (V) und eine Verbindung der Formel (III) oder (IV) in dem inerten Lösungsmittel gelöst beziehungsweise suspendiert. Nach eventuellem Zusatz eines Molekularsiebs oder Aluminiumoxids als Wasserbindemittel wird die organische oder anorganische Base (sofern noch erforderlich) zugesetzt. Anschließend wird zweckmäßigerweise das Reaktionsgemisch eine geraume Zeit vorgerührt, um eventuell noch darin enthaltenes Wasser an das Molekularsieb oder das basische Aluminiumoxid zu binden. Hierbei bildet sich auch die Schiffsche Base, wobei das entstehende Wasser ebenfalls gebunden wird. Nach dieser Voraktivierung wird der vorher entwässerte Hydrierkatalysator hinzugefügt und die hydrogenolytische Umsetzung unter Wasserstoffatmosphäre vorgenommen. Besonders günstig ist dabei ein Wasserstoffüberdruck zwischen 1 und 8 bar.

Das erfindungsgemäße Verfahren ist insbesondere bei reduktiven Aminierungen vorteilhaft, die sauer katalysiert (bei Säureüberschuß) nicht oder nur mit geringen Ausbeuten ablaufen.

Durch die nachfolgenden Beispiele soll das erfindungsgemäße Verfahren näher erläutert werden:

### Beispiel 1:

### N^{α}-[(S)-1-Ethoxycarbonyl-3-phenylpropyl]-L-alanyl-L-prolin

50 mmol (9,3 g) Ala-Pro wurden in 100 ml Ethanol aufgeschlämmt, mit 10 mmol (1 g) Triethylamin, 75 mmol (15,5 g) 2-Oxo-4-phenyl-buttersäureethylester und 18 g Molekularsieb 3 A versetzt und 2 Stunden unter Wasserausschluß gerührt. Nun wurde ein Teelöffel entwässertes Raney-Nickel und 15 g Molekularsieb 3 A zugesetzt, kurz nachgerührt und anschließend 22 Stunden unter 4 bar H₂-Atmosphäre hydriert. Das Reaktionsgemisch wurde filtriert und eingeengt. Umsatz (nach HPLC), bezogen auf eingesetztes Ala-Pro: 95,6 %, Produktausbeute (nach HPLC) : 95 %. Der Rückstand wurde in 250 ml Ethanol aufgenommen und mit konz. HCl auf pH 4,25 eingestellt (hierzu wurde ein Aliquot entnommen, mit Wasser verdünnt und der pH eingestellt, anschließend auf die Gesamtmenge berechnet). Die Lösung wurde unter vermindertem Druck bei 40°C auf 50 g eingeengt, durch Celithe filtriert und auf 100 ml Gesamtvolumen mit Ethylacetat verdünnt. 5,2 g in Ethylacetat + Ethanol gelöste Maleinsäure wurden zugesetzt und mit Enalapril Maleat angeimpft. Das gebildete Kristallisat wurde abgetrennt und getrocknet.
Ausbeute: 20,9 g = 85 % der Theorie,
>99 % SSS-Diastereomer, Schmelzpunkt : 145-146°C, [α]_{D} ²⁵: 42° (c=1 in MeOH).

### Vergleichsversuch:

### N^{α}-[(S)-1-Ethoxycarbonyl-3-phenylpropyl]-L-alanyl-L-prolin

50 mmol (9,3 g) Ala-Pro wurden in 100 ml Ethanol aufgeschlämmt, mit 75 mmol (15,5 g) 2-Oxo-4-phenylbuttersäureethylester und 18 g Molekularsieb 3 A versetzt und 2 Stunden unter Wasserausschluß gerührt. Nun wurde ein Teelöffel entwässertes Raney-Nickel und 15 g Molekularsieb 3 A zugesetzt, kurz nachgerührt und anschließend 22 Stunden unter 4 bar H₂-Atmosphäre hydriert. Das Reaktionsgemisch wurde filtriert und eingeengt. Umsatz (nach HPLC), bezogen auf eingesetztes Ala-Pro: 76,7 %, Produktausbeute (nach HPLC) : 35 %. Der Rückstand wurde in 250 ml Ethanol aufgenommen und mit konz. HCl auf pH 4,25 eingestellt (hierzu wurde ein Aliquot entnommen, mit Wasser verdünnt und der pH eingestellt, anschließend auf die Gesamtmenge berechnet). Die Lösung wurde unter vermindertem Druck bei 40°C auf 50 g eingeengt, durch Celithe filtriert und auf 100 ml Gesamtvolumen mit Ethylacetat verdünnt. 5,2 g in Ethylacetat + Ethanol gelöste Maleinsäure wurden zugesetzt und mit Enalapril Maleat angeimpft. Das gebildete Kristallisat wurde abgetrennt und getrocknet.
Ausbeute: 7,4 g = 30 % der Theorie, > 99 %
SSS-Diastereomer, Schmelzpunkt: 145 - 146°C,
[α]_{D} ²⁵: 42° (c=1 in MeOH).

### Beispiel 2:

### N^{α}-[(S)-1-Ethoxycarbonyl-3-phenylpropyl]-L-alanyl-L-prolin

Es wurde verfahren wie in Beispiel 1, jedoch wurden als Base anstelle von Triethylamin 0,8 moleq Ethanolamin eingesetzt. Umsatz (nach HPLC), bezogen auf Ala-Pro: 85 %, Produktausbeute (nach HPLC) : 85 %, Diastereomerenverhältnis vor Kristallisation:
SSS:RSS-Isomer = 88:12.

### Beispiel 3:

### N^{α}-[(S)-1-Ethoxycarbonyl-3-phenylpropyl]-N^{ε}-trifluoracetyl-L-lysyl-L-prolin

150 mmol H-N^{ε}-TFA-Lys-Pro-H (50,9 g) wurden in 120 ml n-Butanol aufgenommen und im Vakuum ca. 50 ml n-Butanol abdestilliert. Die Lösung wurde mit 600 ml Ethanol verdünnt. Es wurden 22,5 g Molekularsieb 3 A, 34 g 2-Oxo-4-phenyl-buttersäure-ethylester und 30 mmol Triethylamin (4,2 ml) zugesetzt und 2 Stunden unter Wasserausschluß gerührt. Nun wurden drei Teelöffel entwässertes Raney-Nickel zugesetzt und 4 Stunden bei 4 bar H₂-Atmosphäre gerührt. Nach dieser Zeit wurde entspannt, weitere 12,4 g 2-Oxo-4-phenyl-buttersäure ethylester zugesetzt und 2 Stunden nachgerührt. Nun wurden wieder 4 bar H₂ aufgedrückt und bei diesem Druck weitere 4 Stunden hydriert. Der Katalysator wurde abgetrennt (er kann direkt wiederverwendet werden) und die Lösung eingedampft. Umsatz (nach HPLC), bezogen auf N^{ε}-TFA-Lys-Pro: ca. 95 %,
Produktausbeute (nach HPLC) : 90 %, Diastereomerenverhältnis: SSS:RSS-Isomer = 95:5. Der Rückstand wurde in 450 ml Wasser und 450 ml 1,1,1-Trichlorethan bei 5°C suspendiert, in der Wasserphase wurde mit 50%iger NaOH ein pH-Wert von 9,6 eingestellt und die Phasen wurden gut durchmischt. Die organische Phase wurde abgetrennt und die wässrige Produktphase mit 100 ml 1,1,1-Trichlorethan nachextrahiert. Bei 5°C wurde nun in der wässrigen Phase ein pH-Wert von 4,6 eingestellt und diese 2x mit 400 ml 1,1,1-Trichlorethan extrahiert. Die organische Phase wurde eingedampft und in 250 ml Methyl-tert.Butylether aufgenommen.
Die Lösung wurde auf 0°C abgekühlt und angeimpft.
Ausbeute: 48 g (60 % der Theorie), Gehalt an SSS-Diastereomer: >99 %, [α]_{D} ²⁰:-25,5° [(c=1 in MeOH/1N HCl (1:1)].

### Beispiel 4:

### N^{α}-[(S)-1-Ethoxycarbonyl-3-phenylpropyl]-N^{ε}-trifluor-acetyl-L-lysyl-L-prolin

Es wurde verfahren wie im Beispiel 4, jedoch wurde als Base anstelle von Triethylamin 0,2 moleq Tributylamin eingesetzt.
Umsatz (nach HPLC, bezogen auf N^{ε} -TFA-Lys-Pro): 85 %,
Produktausbeute (nach (HPLC) : 75 %,
Diastereomerenverhältnis vor Kristallisation:
SSS:RSS-Diastereomer= 96,4:3,6.

### Beispiel 5:

### N^{α}-[(S)-1-Ethoxycarbonyl-3-phenylpropyl]-N^{ε}-trifluoracetyl-L-lysyl-L-prolin

Es wurde verfahren wie im Beispiel 4, jedoch wurde als Base anstelle von Triethylamin 0,8 moleq Ethanolamin eingesetzt.
Umsatz (nach HPLC, bezogen auf N^{ε}-TFA-Lys-Pro): 75 %,
Produktausbeute (nach HPLC) : 65 %,
Diastereomerenverhältnis vor Kristallisation:
SSS:RSS-Diastereomer= 93:7

### Beispiel 6:

### N^{α}-[(S)-1-Ethoxycarbonyl-3-phenylpropyl]-N^{ε}-trifluoracetyl-L-lysyl-L-prolin

Es wurde verfahren wie im Beispiel 4, jedoch wurde als Base anstelle von Triethylamin 0,2 moleq L-Prolinol eingesetzt.

Umsatz (HPLC) bezogen auf N^{ε}-TFA-Lys-Pro: 85 %, Produktausbeute (HPLC): 65 %, Diastereomerenverhältnis vor Kristallisation: SSS:RSS-Diastereomer= 95:5.

### Beispiel 7:

### N^{α}-[(S)-1-Ethoxycarbonyl-3-phenylpropyl]-N^{ε}-trifluoracetyl-L-lysyl-L-prolin

Es wurden verfahren wie im Beispiel 4, jedoch wurde als Base anstelle von Triethylamin 0,2 moleq (1R,2S)-(-)-Ephedrin eingesetzt. Umsatz (nach HPLC, bezogen auf N^{ε}-TFA-Lys-Pro) : 84 %, Produktausbeute (nach (HPLC) : 70 %, Diastereomerenverhältnis vor Kristallisation: SSS:RSS-Diastereomer= 96,7:3,3.

### Beispiel 8:

### N^{α}-[(S)-1-Ethoxycarbonyl-3-phenylpropyl]-N^{ε}-trifluoracetyl-L-lysyl-L-prolin

Es wurde verfahren wie im Beispiel 4, jedoch wurde als Base 0,1 moleq Triethylamin eingesetzt.
Umsatz (nach HPLC, bezogen auf N^{ε}-TFA-Lys-Pro) : 75 %, Produktausbeute (HPLC): 60 %, Diastereomerenverhältnis vor Kristallisation: SSS:RSS-Diastereomer= 97,7:2,3.

### Beispiel 9:

### N^{α}-[(S)-1-Ethoxycarbonyl-3-phenylpropyl]-N^{ε}-trifluoracetyl-L-lysyl-L-prolin

Es wurde verfahren wie im Beispiel 4, jedoch wurde anstatt Molekularsieb und Base, 25 g basisches aktiviertes Aluminiumoxid eingesetzt.
Umsatz (nach HPLC, bezogen auf N^{ε}-TFA-Lys-Pro) : 95 %,
Produktausbeute (HPLC): 80 %, Diastereomerenverhältnis vor Kristallisation: SSS:RSS-Diastereomer= 95,5:4,5.

### Beispiel 10:

### N^{α}-[(S)-1-Ethoxycarbonyl-3-phenylpropyl]-N^{ε}-trifluor-acetyl-L-lysyl-L-prolin

Es wurde verfahren wie im Beispiel 4, jedoch wurde als Katalysator anstelle von Raney-Nickel Palladium 1 % auf Kohle eingesetzt.
Umsatz (nach HPLC, bezogen auf N^{ε} -TFA-Lys-Pro) : 90 %, Produktausbeute (nach HPLC): 80 %,
Diastereomerenverhältnis vor Kristallisation:
SSS:RSS-Diastereomer= 62:38.

### Beispiel 11:

### N^{α}-[(S)-1-Ethoxycarbonyl-3-phenylpropyl]-L-alanin

250 mmol L-Alaninbenzylester p-Toluolsulfonsäuresalz (88 g) und 312,5 mmol 2-Oxo-4-phenyl-buttersäureethylester (65 g) wurden in 500 ml Cyclohexan suspendiert. Nach Zusatz von 250 mmol N-Methylmorpholin (25,3 g) wurde unter Wasserabscheidung bis zum Ende der Reaktion azeotrop destilliert. Nach dem Abkühlen wurde von Salzen abfiltriert, eingedampft und in 400ml wasserfreiem Methanol aufgenommen. Nach Zusatz von 25 mmol N-Methylmorpholin (1 g) und 3 Teelöffeln entwässertem Raney-Nickel wurde bei 4 bar Wasserstoffüberdruck und Raumtemperatur bis zum Ende der Wasserstoffaufnahme hydriert. Es wurde vom Katalysator abfiltriert, mit warmem Methanol nachgewaschen und eingedampft. Der Rückstand wurde bei 5°C und pH 9,6 zwischen Wasser und 1,1,1-Trichlorethan verteilt. Die Wasserphase wurde anschließend bei pH 3 und 5°C mit 1,1,1-Trichlorethan extrahiert und die organische Phase eingedampft. Der Rückstand wurde aus Ethanol oder Aceton in der Kälte kristallisiert.
Ausbeute: 1. Fraktion: 35 g; 2. Fraktion: 12 g
Schmelzpunkt: 143-145°C,

## Patentansprüche

1. Reduktive Aminierung einer Aminosäure oder eines Aminosäurederivates mit einer α-Ketosäure oder einem α-Ketosäurederivat in einem inerten Lösungsmittel in Gegenwart eines Hydrierkatalysators und Hydriermittels sowie gegebenenfalls unter Wasserentfernung, wobei die reduktive Aminierung die Schritte der Iminbildung und Reduktion zum Amin umfaßt,
**dadurch gekennzeichnet**,
daß man die reduktive Aminierung während der Iminbildung und der Reduktion zum Amin in Gegenwart eines Basenüberschusses vornimmt.

2. Reduktive Aminierung nach Anspruch 1,
**dadurch gekennzeichnet**,
daß die Aminosäure eine α-Aminosäure ist.

3. Reduktive Aminierung nach Anspruch 1 oder 2,
**dadurch gekennzeichnet,**
daß das Aminosäurederivat ein Ester ist.

4. Reduktive Aminierung nach Anspruch 1 oder 2,
**dadurch gekennzeichnet,**
daß das Aminosäurederivat ein Amid ist.

5. Reduktive Aminierung nach Anspruch 4,
**dadurch gekennzeichnet,**
daß das Amid ein Peptid ist.

6. Reduktive Aminierung nach Anspruch 3 oder 5,
**dadurch gekennzeichnet**,
daß eine Verbindung der Formel 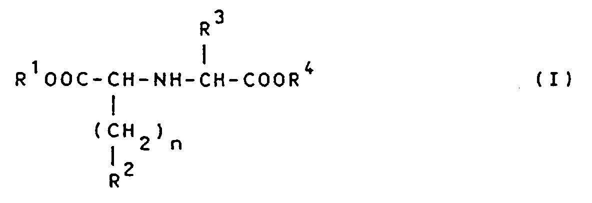 in welcher
R¹ Wasserstoff, Methyl, Ethyl oder Benzyl,
R Wasserstoff, C₁-C₄-Alkyl, Phenyl oder Phenyl-[C₁-C₄-alkyl]
R³ Wasserstoff, Heteroalkyl- oder C₁-C₄-Alkyl-, Benzyl-, 4'-Hydroxybenzyl-, Acylamino-[C₁-C₅]-alkyl-, tert. Butyloxycarbonylamino-[C₁-C₅]-alkyl-, Benzyloxycarbonylamino-[C₁-C₅]-alkyl-
R⁴ H oder eine Esterschutzgruppe,
n 0, 1 oder 2
bedeuten, durch hydrogenolytische Umsetzung einer Verbindung der Formel 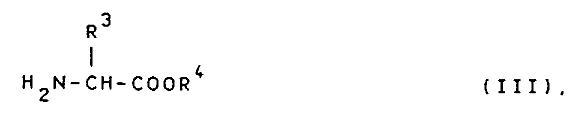 in der R³ und R⁴ eine der bereits angegebenen Bedeutungen aufweisen,
mit einer Verbindung der Formel
R-(CH₂)ₙ-CO-COOR¹ (V),
in der R¹, R und n eine der bereits angegebenen Bedeutungen aufweisen, erhalten wird.
oder daß eine Verbindung der Formel 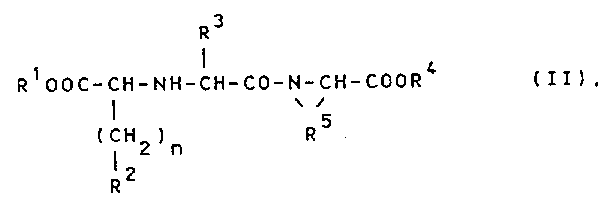 in welcher R¹, R, R³, R⁴ und n die oben angegebene Bedeutung haben,
R⁵ zusammen mit den sie tragenden Atomen ein heterocyclisches mono- oder bicyclisches Ringsystem mit 5 bis 10 Ringgliedern bedeutet,
durch hydrogenolytische Umsetzung einer Verbindung der Formel 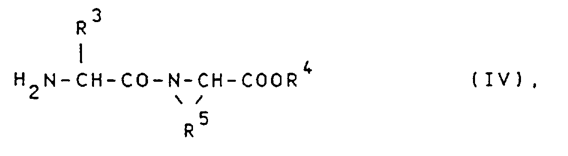 in der R³ bis R⁵ eine der bereits angegebenen Bedeutungen aufweisen, mit einer Verbindung der Formel
R-(CH₂)ₙ-CO-COOR¹ (V),
in der R¹, R und n eine der bereits angegebenen Bedeutungen aufweisen, erhalten wird.

7. Verfahren nach einem der vorhergehenden Ansprüche,
**dadurch gekennzeichnet,**
daß man als organische Base Mono-, Di- oder Triethanolamin, Trimethylamin, Triethylamin, Tripropylamin, Tributylamin, N-Methylmorpholin, L- oder D-Prolinol, L- oder D-Valinol, (-)- oder (+)-Ephedrin, (-)- oder (+)-Norephedrin einsetzt.

8. Verfahren nach einem der Ansprüche 1 - 6,
**dadurch gekennzeichnet,**
daß man als anorganische Base K₂CO₃, Na₂CO₃, KHCO₃, NaOAc, NaHCO₃, oder basisches Aluminiumoxid einsetzt.

9. Verfahren nach einem der vorhergehenden Ansprüche,
**dadurch gekennzeichnet,**
daß man die Base in einer Menge von 0,01 bis 5, vorzugsweise 0,05 - 2 und insbesondere bei organischen Basen besonders bevorzugt 0,08 - 0,6 Moläquivalenten, jeweils bezogen auf die eingesetzte Verbindung der Formel (III) oder (IV), einsetzt.

10. Verfahren nach Anspruch 8 oder 9,
**dadurch gekennzeichnet**,
daß man das basische Aluminiumoxid in einer Menge von 2 g/mol bis 400 g/mol, bezogen auf die eingesetzte Verbindung der Formel (III) oder (IV), einsetzt.

11. Verfahren nach einem der Ansprüche 1 - 6,
**dadurch gekennzeichnet**,
daß man basisches Aluminiumoxid als Wasserbindemittel zur Wasserentfernung einsetzt.

12. Verfahren nach Anspruch 11,
**dadurch gekennzeichnet**,
daß man das basische Aluminiumoxid in einer Menge von 50 g/mol bis 500 g/mol, bezogen auf die eingesetzte Verbindung der Formel (III) oder (IV) einsetzt.

## Claims

1. Reductive amination of an amino acid or of an amino acid derivative using an α-keto acid or an α-keto acid derivative in an inert solvent in the presence of a hydrogenating catalyst and hydrogenating agent, optionally with the removal of water, with the reductive amination comprising the steps of imine formation and reduction to the amine,
characterised in that
the reductive amination, in the course of the imine formation and the reduction to the amine, is carried out in the presence of an excess of base.

2. Reductive amination according to claim 1,
characterised in that
the amino acid is an α-amino acid.

3. Reductive amination according to claim 1 or 2,
characterised in that
the amino acid derivative is an ester.

4. Reductive amination according to claim 1 or 2,
characterised in that
the amino acid derivative is an amide.

5. Reductive amination according to claim 4,
characterised in that
the amide is a peptide.

6. Reductive amination according to claim 3 or 5,
characterised in that
a compound of the formula 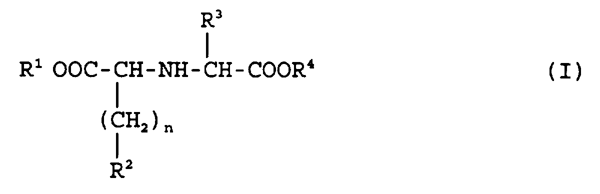 wherein
R¹ signifies hydrogen, methyl, ethyl or benzyl,
R signifies hydrogen, C₁-C₄-alkyl, phenyl or phenyl-[C₁-C₄-alkyl],
R³ signifies hydrogen, heteroalkyl or C₁-C₄-alkyl, benzyl, 4'-hydroxybenzyl, acylamino- [C₁-C₅] -alkyl, tert.-butyloxycarbonylamino- [C₁-C₅] -alkyl, benzyloxycarbonylamino- [C₁-C₅]-alkyl,
R⁴ signifies H or an ester-protecting group,
n is 0, 1 or 2,
is obtained by hydrogenolytic conversion of a compound of the formula 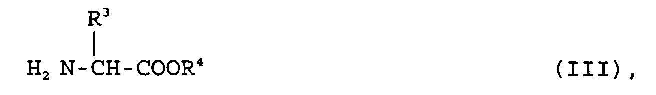 wherein R³ and R⁴ have one of the meanings already given, together with a compound of the formula
R - (CH₂)ₙ -CO-COOR¹ (V),
wherein R¹, R and n have one of the meanings already given, or that a compound of the formula 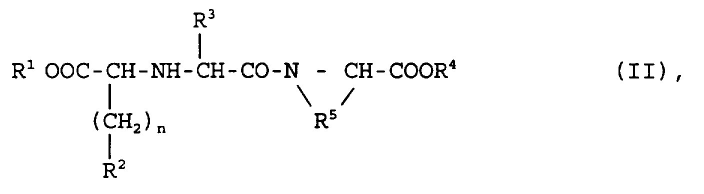 wherein R¹, R, R³, R⁴ and n have the meanings given above,
R⁵ together with the atoms carrying it signifies a heterocyclic monocyclic or bicyclic ring system having 5 to 10 ring members,
is obtained by hydrogenolytic conversion of a compound of the formula 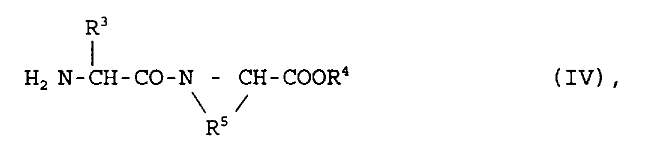 wherein R³ to R⁵ have one of the meanings already given, together with a compound of the formula
R - (CH₂)ₙ -CO-COOR¹ (V),
wherein R¹, R and n have one of the meanings already given.

7. Method according to one of the preceding claims,
characterised in that
the organic base used is mono-, di- or triethanolamine, trimethylamine, triethylamine, tripropylamine, tributylamine, n-methylmorpholine, L- or D-prolinol, L- or D-valinol, (-)- or (+)-ephedrine, (-)- or (+)-norephedrine.

8. Method according to one of claims 1 to 6,
characterised in that
the inorganic base used is K₂CO₃, Na₂CO₃, KHCO₃, NaOAc, NaHCO₃, or basic aluminium oxide.

9. Method according to one of the preceding claims,
characterised in that
the base is used in a quantity of from 0.01 to 5 molar equivalents, preferably 0.05 to 2 molar equivalents, and particularly when organic bases are used, particularly preferably of from 0.08 to 0.6 molar equivalents, in each case referred to the compounds used having the formulae (III) or (IV).

10. Method according to claim 8 or 9,
characterised in that
the basic aluminium oxide is used in a quantity of from 2 g/mol to 400 g/mol, referred to the compounds used having the formulae (III) or (IV).

11. Method according to one of claims 1 to 6,
characterised in that
the basic aluminium oxide is used as a water-binding agent for the removal of water.

12. Method according to claim 11,
characterised in that
the basic aluminium oxide is used in a quantity of from 50 g/mol to 500 g/mol, referred to the compounds used having the formulae (III) or (IV).

## Revendications

1. Amination réductive d'un amino-acide ou d'un dérivé d'amino-acide à l'aide d'un acide α-cétonique ou d'un dérivé d'acide α-cétonique dans un solvant inerte en présence d'un catalyseur d'hydrogénation et d'un agent d'hydrogénation, et éventuellement avec élimination d'eau, l'amination réductive englobant les étapes de formation d'imine et de réduction en amine, caractérisée en ce qu'on procède à l'amination réductive au cours de la formation d'imine et de la réduction en amine en présence d'une base en excès.

2. Amination réductive selon la revendication 1, caractérisée en ce que l'amino-acide est un α-amino-acide.

3. Amination réductive selon la revendication 1 ou 2, caractérisée en ce que le dérivé d'amino-acide est un ester.

4. Amination réductive selon la revendication 1 ou 2, caractérisée en ce que le dérivé d'amino-acide est un amide.

5. Amination réductive selon la revendication 4, caractérisée en ce que l'amide est un peptide.

6. Amination réductive selon la revendication 3 ou 5, caractérisée en ce qu'on obtient un composé de formule 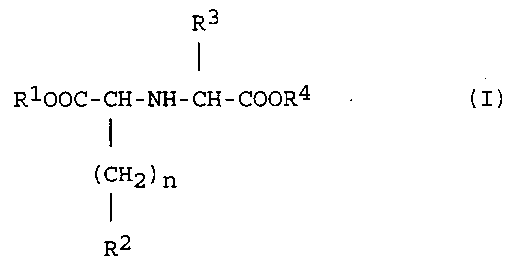 dans laquelle
R¹ représente un atome d'hydrogène, un groupe méthyle, un groupe éthyle ou un groupe benzyle,
R représente un atome d'hydrogène, un groupe alkyle en C₁-C₄, un groupe phényle ou un groupe phénylalkyle en C₁-C₄,
R³ représente un atome d'hydrogène, un groupe hétéroalkyle ou alkyle en C₁-C₄, un groupe benzyle, un groupe 4'hydroxybenzyle, un groupe acylaminoalkyle en C₁-C₅, un groupe tert.-butyloxycarbonylaminoalkyle en C₁-C₅, un groupe benzyloxycarbonylaminoalkyle en C₁-C₅,
R⁴ représente H ou un groupe protecteur du groupe ester,
n représente 0, 1 ou 2,
par mise en réaction hydrogénolytique d'un composé de formule 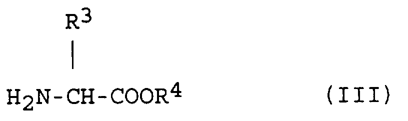 dans laquelle R³ et R⁴ présentent une des significations déjà indiquées,
avec un composé de formule
R-(CH₂)ₙ-CO-COOR¹ (V)
dans laquelle R¹, R et n présentent une des significations déjà indiquées;
ou bien en ce qu'on obtient un composé de formule 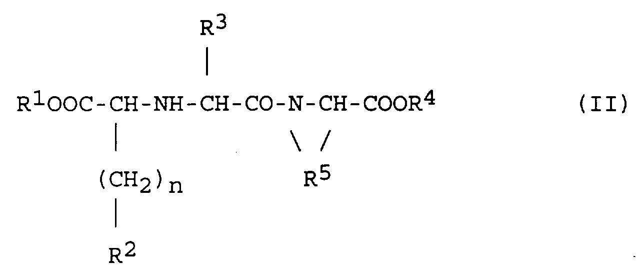 dans laquelle R¹, R, R³, R⁴ et n ont la signification indiquée ci-dessus,
R⁵ représente, conjointement avec les atomes qui le portent, un système hétérocyclique mono- ou bicyclique contenant de 5 à 10 termes cycliques,
par mise en réaction hydrogénolytique d'un composé de formule 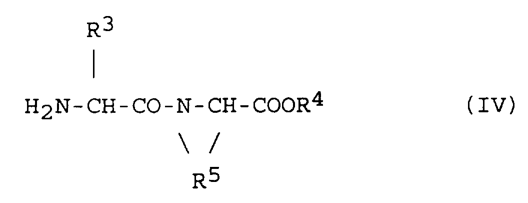 dans laquelle R³ à R⁵ présentent une des significations déjà indiquées, avec un composé de formule
R-(CH₂)ₙ-CO-COOR¹ (V)
dans laquelle R¹, R et n présentent une des significations déjà indiquées.

7. Procédé selon l'une quelconque des revendications précédentes, caractérisé en ce qu'on met en oeuvre, comme base organique, la mono-, la di- ou la triéthanolamine, la triméthylamine, la triéthylamine, la tripropylamine, la tributylamine, la N-méthylmorpholine, le L- ou D-prolinol, le L- ou D-valinol, la (-) ou (+)-éphédrine, la (-) ou (+)-noréphédrine.

8. Procédé selon l'une quelconque des revendications 1-6, caractérisé en ce que, comme base inorganique, on met en oeuvre K₂CO₃, Na₂CO₃, KHCO₃, NaOAc, NaHCO₃ ou encore de l'oxyde d'aluminium basique.

9. Procédé selon l'une quelconque des revendications précédentes, caractérisé en ce qu'on met en oeuvre la base en une quantité de 0,01 à 5, de préférence de 0,05 à 2, en particulier dans le cas de bases organiques, de manière particulièrement préférée de 0,08 à 0,6 équivalent molaire, chaque fois rapporté au composé de formule (III) ou (IV) mis en oeuvre.

10. Procédé selon la revendication 8 ou 9, caractérisé en ce qu'on met en oeuvre l'oxyde d'aluminium basique en une quantité de 2 g/mole à 400 g/mole, rapportée au composé de formule (III) ou (IV).

11. Procédé selon l'une quelconque des revendications 1 à 6, caractérisé en ce qu'on met en oeuvre de l'oxyde d'aluminium basique comme agent liant l'eau pour éliminer l'eau.

12. Procédé selon la revendication 11, caractérisé en ce qu'on met en oeuvre l'oxyde d'aluminium basique en une quantité de 50 g/mole à 500 g/mole, rapportée au composé de formule (III) ou (IV) mis en oeuvre.
